(19) Europäisches Patentamt
European Patent Office
Office européen des brevets

(11) EP 3 156 010 A1

(12) EUROPEAN PATENT APPLICATION
published in accordance with Art. 153(4) EPC

(43) Date of publication:
19.04.2017 Bulletin 2017/16

(51) Int Cl.:
A61F 2/70 (2006.01)          A61F 2/72 (2006.01)
B25J 11/00 (2006.01)

(21) Application number: 15807079.7

(22) Date of filing: 12.06.2015

(86) International application number:
PCT/JP2015/067034

(87) International publication number:
WO 2015/190598 (17.12.2015 Gazette 2015/50)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 13.06.2014 JP 2014122482

(71) Applicants:
• Cyberdyne Inc.
Tsukuba-shi, Ibaraki 305-0818 (JP)

• University of Tsukuba
Tsukuba-shi, Ibaraki 305-8577 (JP)

(72) Inventor: SANKAI, Yoshiyuki
Tsukuba-shi
Ibaraki 305-8577 (JP)

(74) Representative: Hoffmann Eitle
Patent- und Rechtsanwälte PartmbB
Arabellastraße 30
81925 München (DE)

(54) **WORN-PROSTHETIC-LIMB MOVEMENT ASSISTANCE DEVICE AND WORN MOVEMENT ASSISTANCE DEVICE**

(57) Provided are a prosthesis-mounted action-assist device and a wearable action-assist device that enable step-over-step stair ascending and descending and achieve smooth switching between tasks in accordance with a motion of a wearer. The prosthesis-mounted action-assist device assists an action of a knee disposed between an upper leg frame and a lower leg frame of a prosthetic leg. The device includes an actuator applying power to the prosthetic leg, an absolute angle sensor detecting a hip angle relative to a vertical direction, an angle sensor detecting a knee angle, a floor reaction force sensor detecting a floor reaction force to a wearer, a data storage unit storing reference parameters of phases of tasks, and a control unit comparing the hip angle, the knee angle, and the floor reaction force with the reference parameters stored in the data storage unit to estimate a phase of a task of the wearer and generating an autonomous command signal for causing the actuator to produce power for the phase.

Fig. 1

**Description**

Technical Field

The present invention relates to a prosthesis-mounted action-assist device that assists an action of a prosthesis wearer and a wearable action-assist device.

Background Art

Legs are important body parts for achieving moving actions, such as walking on level ground and ascending or descending stairs, in daily life. The loss of a leg due to an accident or the like leads to a reduction in ability to move. Leg amputations are classified broadly into two categories: a below-knee amputation at any point between the ankle and the knee and an above-knee amputation at any point between the knee and the hip joint.

A below-knee amputation allows a knee to be preserved. An amputee can perform actions, such as walking and ascending and descending stairs, by using a below-knee prosthetic leg.

An above-knee amputation causes the need for supplementing the lost knee with an above-knee prosthetic leg. A typical above-knee prosthetic leg is a passive prosthetic leg including a prosthetic knee, called a knee joint, having spring or damper characteristics. A recently developed wearable action-assist device (refer to Patent Literature 1, for example) includes driving means that drives a prosthetic joint and voluntary control means that causes the driving means based on a biological signal detected from a thigh of a wearer to produce power based on an intention of the wearer. The device classifies action patterns of the wearer as tasks each including a series of phases (minimum motion units). The device further includes autonomous control means and causes this means to estimate a phase of a task of the wearer based on physical quantities, such as an angle of rotation of a knee portion and a floor reaction force, and causes the driving means to produce power for the phase.

PTL 1: Japanese Unexamined Patent Application Publication No. 2009-60946

Summary of Invention

The above-described related-art prosthetic legs relatively easily enable walking on level ground. It is, however, difficult for a wearer of such a prosthetic leg to ascend and descend stairs one at a time, or in a step-over-step manner like an able-bodied person. In addition, since these prosthetic legs cannot determine an intention of the wearer, they fail to perform smooth switching, for example, from level walking to stair ascending or descending or from stair ascending or descending to level walking. It is difficult for a wearer of such a prosthetic leg to achieve walking similar to that of an able-bodied person.

The present invention aims to provide a prosthesis-mounted action-assist device and a wearable action-assist device that enable step-over-step stair ascending and descending and achieve smooth switching between three tasks, namely, level walking, stair ascending, and stair descending, in accordance with a motion of a wearer.

According to one aspect of the present invention, there is provided a prosthesis-mounted action-assist device that assists an action of a knee disposed between an upper leg frame and a lower leg frame of a prosthetic leg, the device including an actuator disposed outside the knee and connected to the upper leg frame and the lower leg frame, the actuator applying power to the prosthetic leg, an absolute angle sensor detecting a hip angle relative to a vertical direction, an angle sensor disposed in the actuator, the angle sensor detecting a knee angle, a floor reaction force sensor detecting a floor reaction force to a wearer, a data storage unit storing reference parameters of minimum motion units (phases) of action patterns of the wearer classified as tasks, each task including a series of phases, and a control unit comparing the hip angle, the knee angle, and the floor reaction force with the reference parameters stored in the data storage unit to estimate a phase of a task of the wearer and generating an autonomous command signal for causing the actuator to produce power for the phase.

According to one aspect of the present invention, the control unit calculates a target knee angle based on lengths of the upper and lower leg frames by using inverse kinematics to allow a toe of the prosthetic leg to pass over a step having a predetermined height in a flexion phase of a stair ascending task, and generates the autonomous command signal based on a difference between the target knee angle and the knee angle detected by the angle sensor.

According to one aspect of the present invention, the control unit calculates a target knee angle based on the hip angle to cause a bottom of a foot portion to be in a flat position in a foot flat phase of a stair ascending task, and generates the autonomous command signal based on a difference between the target knee angle and the knee angle detected by the angle sensor.

According to one aspect of the present invention, the control unit causes transition from the flexion phase to a foot flat phase when the knee angle detected by the angle sensor reaches the target knee angle.

According to one aspect of the present invention, the control unit generates the autonomous command signal based on a product of a gain and a difference between the knee angle detected by the angle sensor and a knee angle in a fully extended position in a stance phase of a stair descending task or a level walking task, and a gain for flexion is greater than a gain for extension.

According to one aspect of the present invention, the control unit calculates a hip angular velocity based on the hip angle in a swing phase of a level walking task, and generates the autonomous command signal based on a product of a gain and a hip angular velocity to be obtained a predetermined time before.

According to one aspect of the present invention, the control unit performs switching between a level walking task and a stair ascending task based on the hip angle, the knee angle, and the floor reaction force.

According to one aspect of the present invention, the control unit performs switching from the level walking task to the stair ascending task based on a floor reaction force to a healthy leg, a floor reaction force to the prosthetic leg, a vertical acceleration obtained from the absolute angle sensor, and the hip angle.

According to one aspect of the present invention, the control unit performs switching from the stair ascending task to the level walking task when a floor reaction force to a healthy leg is at or above a predetermined value and the knee is moving in a flexion direction.

According to one aspect of the present invention, the control unit performs switching from the stair ascending task to the level walking task when a floor reaction force to a healthy leg is at or below a predetermined value and the knee is moving in an extension direction.

According to one aspect of the present invention, the device further includes a biological signal sensor detecting a biological signal in a thigh of the wearer, and the control unit causes transition from a flexion phase to a bottom flat phase when the biological signal detected from the thigh during extension by the biological signal sensor has a strength at or above a predetermined value in a stair ascending task.

According to one aspect of the present invention, the control unit generates a voluntary command signal for causing the actuator to produce power based on the biological signal, and combines the voluntary command signal and the autonomous command signal to generate a combined command signal, and the actuator is driven by a drive current generated based on the combined command signal.

According to one aspect of the present invention, the data storage unit stores a combination ratio of the voluntary command signal to the autonomous command signal set for each of the phases of the tasks, and the control unit combines the voluntary command signal and the autonomous command signal in accordance with the combination ratio for an estimated phase to generate a combined command signal.

According to one aspect of the present invention, there is provided a wearable action-assist device including an upper leg frame having a first end connected to a socket to be attached to a thigh of a wearer, a knee portion having a first side connected to the upper leg frame, a lower leg frame having a first end connected to a second side of the knee portion, a foot portion connected to a second end of the lower leg frame, an actuator disposed in the knee portion, the actuator producing a driving force to rotate the upper leg frame or the lower leg frame, an angle sensor disposed in the knee portion, the angle sensor detecting a knee angle, an absolute angle sensor disposed in the upper leg frame, the absolute angle sensor detecting a hip angle relative to a vertical direction, a floor reaction force sensor disposed in the foot portion, the floor reaction force sensor detecting a floor reaction force to the wearer, a data storage unit storing reference parameters of minimum motion units (phases) of action patterns of the wearer classified as tasks, each task including a series of phases, and a control unit comparing the hip angle, the knee angle, and the floor reaction force with the reference parameters stored in the data storage unit to estimate a phase of a task of the wearer, and generating an autonomous command signal for causing the actuator to produce power for the phase.

Advantageous Effects of Invention

According to the present invention, step-over-step stair ascending and descending can be performed. In addition, switching between three tasks, namely, level walking, stair ascending, and stair descending, can be smoothly performed in accordance with a motion of a wearer to achieve walking similar to that of an able-bodied person.

Brief Description of Drawings

[Fig. 1] Fig. 1 is an external view of a wearable action-assist device according to an embodiment of the present invention.

[Fig. 2] Fig. 2 is a block diagram illustrating a control system of the wearable action-assist device according to the embodiment.

[Fig. 3] Fig. 3 is a diagram illustrating a series of motions of human level walking.

[Figs. 4] Fig. 4a is a diagram illustrating an exemplary step-over-step stair ascending action and Fig. 4b is a diagram illustrating an exemplary step-by-step stair ascending action.

[Fig. 5] Fig. 5 is a diagram illustrating a flexion phase, a foot flat phase, and a pull-up phase of the stair ascending action.

[Figs. 6] Fig. 6a is a diagram illustrating a stance phase of a stair descending action and Fig. 6b is a diagram illustrating a swing phase thereof.

[Figs. 7] Figs. 7a to 7c are diagrams illustrating exemplary circumstances in which task switching between a level walking task and a stair ascending task occurs.

[Fig. 8] Fig. 8 is a diagram illustrating parameters obtained during level walking of an able-bodied person.

[Fig. 9] Fig. 9 is a diagram illustrating a path used in a level walking action experiment.

[Fig. 10] Fig. 10 is a graph illustrating speeds as results of the level walking action experiment on an able-bodied person.

[Fig. 11] Fig. 11 is a graph illustrating speeds as results of a stair ascending action experiment on the able-bodied person.

[Fig. 12] Fig. 12 is a graph illustrating speeds as results of a stair descending action experiment on the able-bodied person.

[Fig. 13] Fig. 13 is a diagram illustrating a path that requires a level walking action, the stair ascending action, and the stair descending action.

[Fig. 14] Fig. 14 is a diagram illustrating results of a task switching experiment on the able-bodied person.

[Fig. 15] Fig. 15 includes graphs illustrating results of a biopotential measurement experiment on a leg amputee.

[Fig. 16] Fig. 16 is a diagram illustrating knee angles, hip angles, floor reaction forces to both legs, and phase numbers of one gait cycle in the level walking action experiment on the leg amputee.

[Fig. 17] Fig. 17 is a graph illustrating speeds as results of the level walking action experiment on the leg amputee.

[Fig. 18] Fig. 18 is a diagram illustrating knee angles, hip angles, floor reaction forces to both legs, and phase numbers of one gait cycle in the stair ascending action experiment on the leg amputee.

[Fig. 19] Fig. 19 is a diagram illustrating knee angles, hip angles, floor reaction forces to both legs, and phase numbers of one gait cycle in the stair descending action experiment on the leg amputee.

[Fig. 20] Fig. 20 is a graph illustrating speeds as results of the stair ascending action experiment on the leg amputee.

[Fig. 21] Fig. 21 is a graph illustrating speeds as results of the stair descending action experiment on the leg amputee.

[Fig. 22] Fig. 22 is a diagram illustrating results of the task switching experiment on the leg amputee.

[Fig. 23] Fig. 23 is a schematic diagram of the interior of a structure.

[Fig. 24] Fig. 24 is a graph illustrating the times required to walk in sections of a route in the structure.

Description of Embodiments

Embodiments of the present invention will be described below with reference to the drawings.

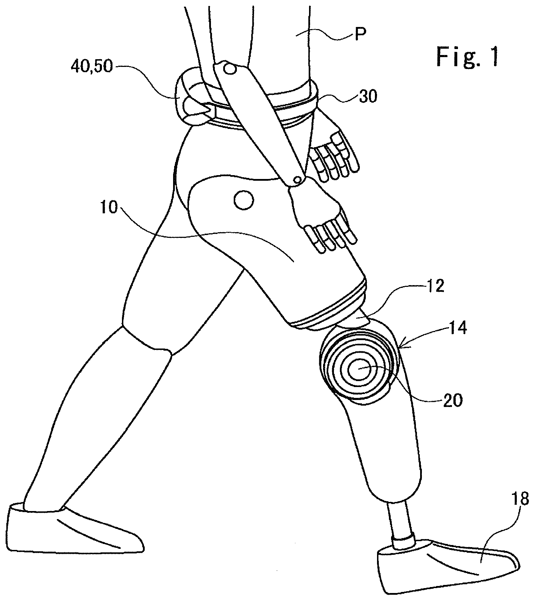

Fig. 1 is an external view of a wearable action-assist device (hereinafter, referred to as an "action-assist device") according to an embodiment of the present invention. Fig. 1 illustrates an exemplary action-assist device attached to an amputated right thigh of a wearer P. The action-assist device includes a socket 10, an upper leg frame 12, a knee portion 14, a lower leg frame 16, and a foot portion 18.

The socket 10 has an insertion recess to fit the right thigh such that the recess is disposed in an inner surface of the socket. The socket 10 is molded of, for example, resin. The socket 10 is attached to the wearer P by, for example, engaging the stump of the amputated right thigh of the wearer P with the insertion recess of the socket 10, removing air between the stump and the socket 10, and applying a negative pressure to the socket 10.

The upper leg frame 12 is connected at a first end to the socket 10 by an attachment (not illustrated) and is connected at a second end to the knee portion 14. The upper leg frame 12 includes an absolute angle sensor that detects an angle formed by a hip joint and the vertical direction. The absolute angle sensor uses an acceleration sensor in low frequency operations or a gyro in high frequency operations to calculate an absolute angle (hip angle) formed by the hip joint (thigh) and the vertical direction.

The knee portion 14 functions as a right knee of the wearer P. The knee portion 14 is connected at a first side to the second end of the upper leg frame 12 and is connected at a second side to a first end of the lower leg frame 16. The knee portion 14 rotatably couples the upper leg frame 12 and the lower leg frame 16. The knee portion 14 includes an actuator 20. The actuator 20 includes an electric motor and directly drives the knee portion 14. Consequently, the knee can be actively flexed and extended. The actuator 20 includes an angle sensor (not illustrated) that detects an angle (flexion angle) of the knee. The angle sensor is, for example, a potentiometer.

The lower leg frame 16 is connected at a second end to the foot portion 18. As the foot portion 18, an energy-storing prosthetic foot member capable of using energy, stored mainly due to an elastic force of a carbon plate during a stance phase, for kick is used. The foot portion 18 includes an insole-shaped floor reaction force sensor (not illustrated). The floor reaction force sensor measures a pressure applied to a floor by the wearer P to obtain a reaction force from the floor to the wearer P. Such a floor reaction force sensor is also attached to the healthy foot (left foot in the case of Fig. 1).

In addition, a biological signal detection sensor (not illustrated) that detects a biopotential (biological signal) in the right thigh is attached to a skin surface of front or rear part of the right thigh of the wearer P.

A waist belt 30 is attached to the waist of the wearer P. A controller 40 that obtains detection results of the sensors to control the actuator 20 and a battery 50 that supplies electric power to the controller 40 and the sensors are mounted on rear part of the waist belt 30.

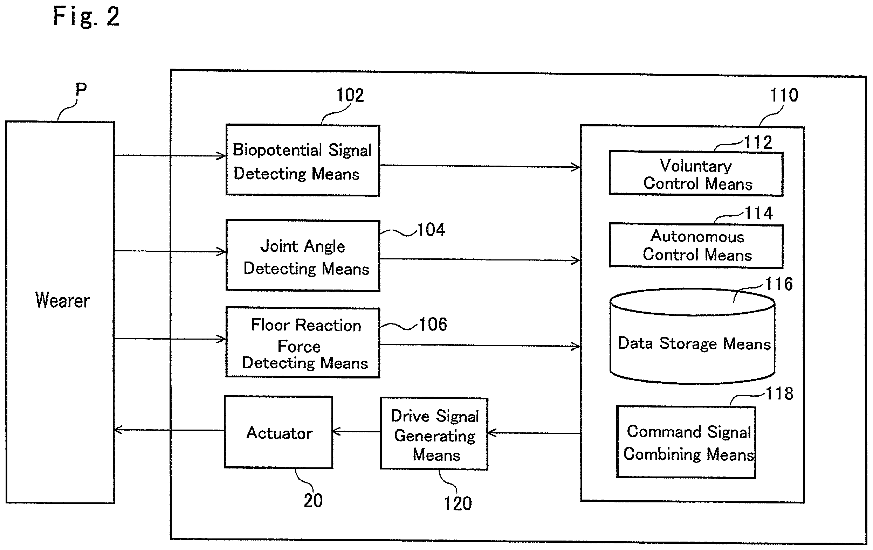

Fig. 2 is a block diagram illustrating a control system of the action-assist device. The action-assist device includes biopotential signal detecting means 102, joint angle detecting means 104, floor reaction force detecting means 106, a control unit 110, drive signal generating means 120, and the actuator 20.

The biopotential signal detecting means 102 includes the above-described biological signal detection sensor. The joint angle detecting means 104 includes the above-described absolute angle sensor and the angle sensor included in the actuator 20. The floor reaction force detecting means 106 includes the above-described floor reaction force sensors.

The control unit 110 includes voluntary control means 112, autonomous control means 114, data storage means 116, and command signal combining means 118, and corresponds to the controller 40 in Fig. 1.

The voluntary control means 112 performs signal processing, including filtering (smoothing) and amplification, on a biopotential signal detected by the biopotential signal detecting means 102. The voluntary control means 112 generates, based on the biopotential signal subjected to the signal processing, a voluntary command signal for causing the actuator 20 to produce power based on an intention of the wearer P.

The data storage means 116 stores a reference parameter database for identifying a phase of a task of the wearer P and information about methods of controlling drive torque of the actuator 20 based on the identified phase. The term "tasks" as used herein refers to classified main action patterns of humans. The term "phases" as used herein refers to a series of minimum motion units constituting a task. Analysis of general human actions reveals that a typical action pattern including the angles of joints and a shift in the center of gravity is determined in each phase. For example, a typical change in angle of each joint and a state of shift in the center of gravity are empirically obtained for each of the phases of many fundamental human actions (tasks) and are then stored into the reference parameter database. The tasks and the phases will be described later.

The autonomous control means 114 compares parameters indicating an action state of the wearer P, for example, joint angles detected by the joint angle detecting means 104 and floor reaction forces detected by the floor reaction force detecting means 106, with reference parameters stored in the data storage means 116 to identify a task and a phase corresponding to an action of the wearer P. The autonomous control means 114 generates an autonomous command signal for causing the actuator 20 to produce power for the identified phase.

The command signal combining means 118 combines a voluntary command signal generated by the voluntary control means 112 and an autonomous command signal generated by the autonomous control means 114 into a combined command signal and outputs the combined command signal to the drive signal generating means 120.

The combination ratio (hybrid ratio) of the voluntary command signal to the autonomous command signal may be preset for each of the phases of the tasks and be stored in the data storage means 116. The hybrid ratio is preset for each of the phases of the tasks so that an action of the wearer P can be assisted without causing uncomfortable feeling. The command signal combining means 118 combines the voluntary command signal and the autonomous command signal at the hybrid ratio for an identified phase to generate a combined command signal.

The combined command signal has a waveform that causes the actuator 20 to produce combined power obtained by combining voluntarily controlled power that varies from the start of an action to the end thereof and autonomously controlled power for each phase.

The drive signal generating means 120 generates a drive signal (drive current) based on a combined command signal and supplies the generated signal to the actuator 20, thus driving the actuator 20. The actuator 20 applies an assist force (power) based on the drive signal to the wearer P.

If the biopotential signal detecting means 102 fails to detect a biopotential signal because, for example, the skin of the wearer P is slick with sweat, generation of a voluntary command signal by the voluntary control means 112 may be omitted and the actuator 20 may be caused to produce power based on an autonomous command signal generated by the autonomous control means 114.

The tasks, the phases, and the methods, performed by the autonomous control means 114, of controlling a command torque to the actuator 20 will now be described. Examples of the tasks as classified actions of the wearer P include a level waking action, a stair ascending action, and a stair descending action.

[Level Walking Action]

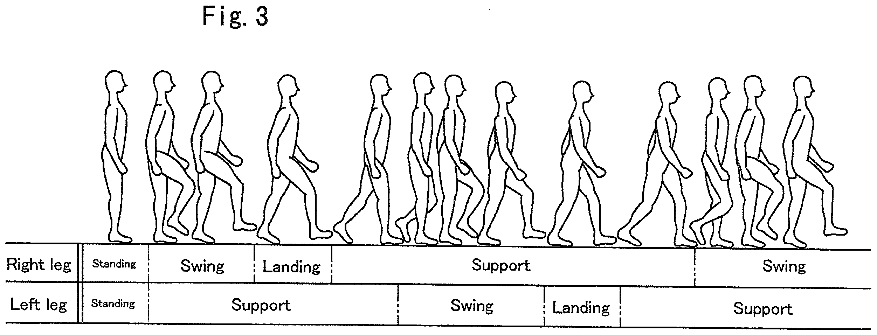

Fig. 3 illustrates a series of motions of human level walking. Human walking states are classified broadly into two categories, a swing phase and a stance phase. The swing phase is a period during which the foot leaves the ground and the lower leg is swung. The stance phase is a period during which the foot is in contact with the ground to support the weight of a person. A level ground walking task includes the swing phase and the stance phase.

In the swing phase, the knee flexes immediately after transition from the stance phase to the swing phase. The hip joint flexes for the next step. At the middle of the stance phase, the knee begins to extend with a time lag. As described above, the lower leg flexes and extends in conjunction with and following the motion of the thigh in the swing phase. In the present embodiment, a command torque for the actuator 20 is calculated based on a hip angular velocity obtained from the absolute angle sensor so that the lower leg moves in conjunction with and following the motion of the thigh in the swing phase. The command torque in the swing phase is given by Expression (1) as follows.

[Math. 1]

$$\tau_{command} = K_{hip} \cdot \dot{\theta}_{hip}(t - T) \cdots (1)$$
$$\begin{cases} K_{hip} = K_{fl} : \left( \dot{\theta}_{hip}(t - T) > 0 \right) \\ K_{hip} = K_{ex} : \left( \dot{\theta}_{hip}(t - T) \leq 0 \right) \end{cases}$$

$\tau_{command}$ is the command torque,
$\dot{\theta}_{hip}(t)$ is the hip angular velocity,
$K_{hip}$ is the gain,
$T$ is the delay time.

The delay time T is, for example, approximately 100 ms. The gains $K_{fl}$ and $K_{ex}$ are different values.

Torque control in the swing phase corresponds to a feed-forward control system. If transition to the swing phase of the level walking task accidentally occurs, therefore, the knee will not be driven as long as the wearer voluntarily flexes or extends the thigh.

Torque control in the stance phase is the same as that in the stair descending action, which will be described later. To prevent unintended knee flexion caused by, for example, stumbling, position control in the stance phase is performed using an angle at a fully extended position as a target angle. At transition from the swing phase to the stance phase, the prosthetic leg in the fully extended position contacts the floor. Such a hardware structure can prevent unintended knee flexion in the stance phase. Torque control in the stance phase is set to be identical to that in the stair descending action, which will be described later. Since stair descending is performed by using the stance phase of the level walking action, the need for switching between tasks can be eliminated, thus simplifying a program.

The level walking action is achieved by repeating the torque control in the swing phase and that in the stance phase described above.

[Stair Ascending Action]

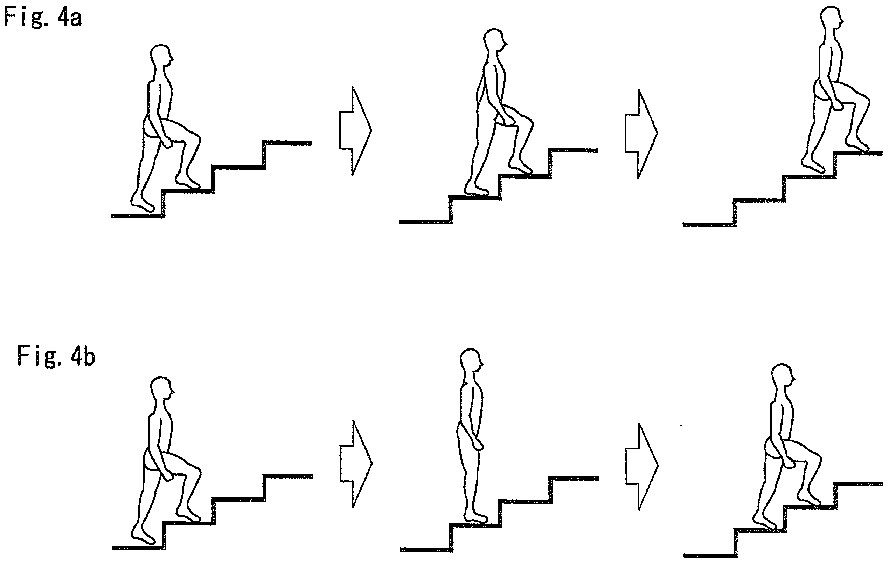

Torque control in the stair ascending action will now be described. The stair ascending action in the present embodiment is an ascending action in the step-over-step manner. Fig. 4a illustrates an exemplary step-over-step stair ascending action. Fig. 4b illustrates an exemplary step-by-step stair ascending action. The term "step-over-step" means a typical manner of ascending or descending stairs one at a time while alternating legs. An able-bodied person ascends or descends stairs in this manner. The term "step-by-step" means a manner of ascending or descending stairs such that a person places both foots on the same stair and then goes up or down by using one leg. A person having a leg problem or an injured leg ascends or descends stairs in this manner.

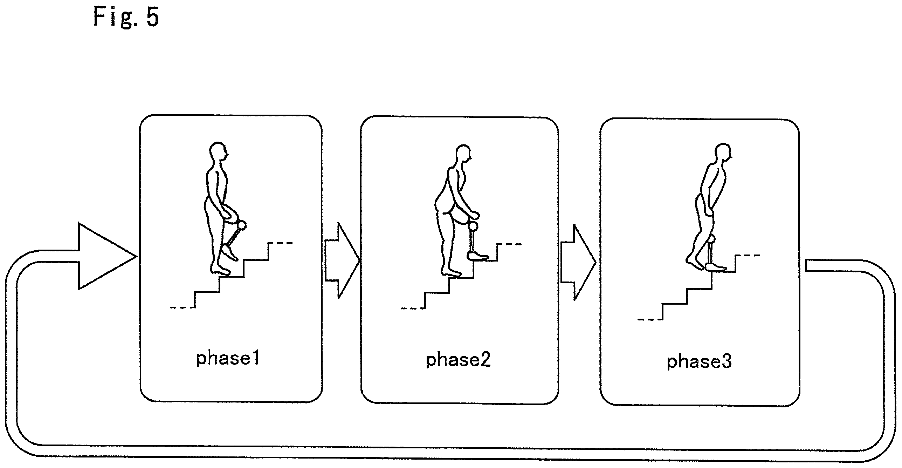

As illustrated in Fig. 5, a stair ascending task includes three phases: a flexion phase (phase 1), a foot flat phase (phase 2), and a pull-up phase (phase 3). A target trajectory is generated for each of the phases, and torque control is performed to follow the trajectories. To generate a trajectory, a minimum jerk trajectory, which is said to be similar to human reaching movement, is used to reproduce smooth human motion.

In the flexion phase (phase 1), the knee is flexed to prevent the wearer from hitting the toe of the foot portion 18 against a riser of the stairs, or from stumbling. According to the Building Standards Law, the dimensions of risers of stairs are determined at or below 230 mm. A target knee angle for the first step is calculated based on the link lengths of upper and lower legs of the wearer P by using inverse kinematics so that the toe can pass over a step corresponding to a riser height of 230 mm. The wearer P flexes the hip joint in synchronization with the flexion of the knee so that the toe of the foot portion 18 does not touch the riser in an extending motion in the foot flat phase (phase 2). When the flexion angle of the knee reaches the target angle, the phase shifts to the foot flat phase (phase 2). Alternatively, when the strength of a biopotential signal from the extended leg detected by the biological signal detection sensor is greater than or equal to a predetermined value, the phase may shift to the foot flat phase (phase 2).

In the foot flat phase (phase 2), the knee is controlled such that the bottom of the foot portion is in flat contact with the ground, or in a flat position. A target knee angle at which the bottom of the foot portion is in the flat position is calculated based on the angle of the thigh obtained from the absolute angle sensor, and torque control is performed such that the flexion angle of the knee reaches the target angle. At completion of the extending motion in the foot flat phase (phase 2), the wearer P puts his or her weight on the foot portion 18 of the action-assist device placed on a tread. When the wearer puts the weight at a predetermined proportion, the phase shifts to the pull-up phase (phase 3).

In the pull-up phase (phase 3), when the action-assist device serves as a supporting leg, torque control is performed using the angle in the fully extended position as a target angle. After the knee is fully extended, when the foot portion 18 of the action-assist device leaves the tread, the phase shifts to the flexion phase (phase 1).

Stair ascending is achieved by repeating the three phases, the flexion phase (phase 1), the foot flat phase (phase 2), and the pull-up phase (phase 3). In each of the phases, the controller is based on PID control. PID gains are determined by trial and error. A command torque for the stair ascending action is given by Expressions (2) and (3) as follows.

[Math. 2]

$$e(t) = \theta(t)_{knee\_ref} - \theta(t)_{knee} \cdots (2)$$

$$\tau_{command} = K_p \cdot e(t) + K_D \cdot \dot{e}(t) + K_I \cdot \int e(t)dt \cdots (3)$$

$\theta(t)_{knee}$ is the knee angle,
$\theta(t)_{knee\_ref}$ is the target knee angle,
$e(t)$ is the difference between the knee angle and the target angle,
$K_p$ is the P gain, $K_D$ is the D gain, and $K_I$ is the I gain.

[Stair Descending Action]

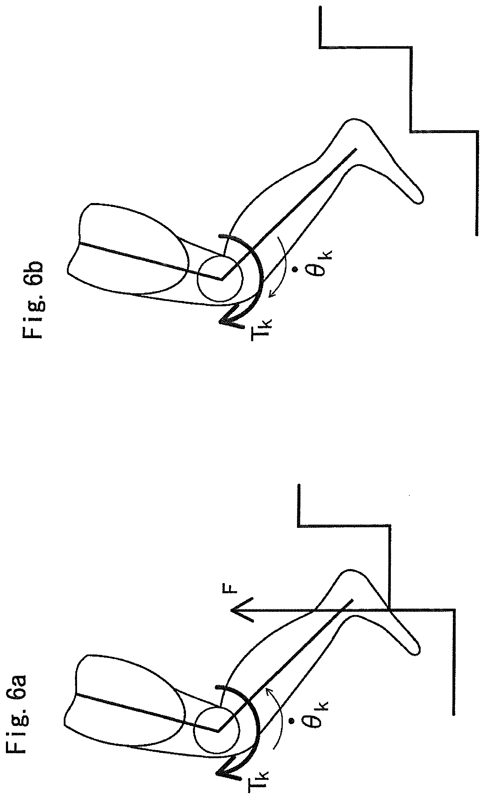

Torque control in the stair descending action will now be described. The stair descending action is a descending action in the step-over-step manner as in the above-described stair ascending action. As illustrated in Figs. 6a and 6b, the stair descending action includes a stance phase (Fig. 6a) and a swing phase (Fig. 6b).

In the stance phase of Fig. 6a, the knee is gradually flexed while supporting the weight of the wearer. At this time, the knee is flexed due to an external force F, and a torque $\tau_k$ for supporting the wearer's weight is output in an extension direction opposite to the direction of the external force F.

In the swing phase of Fig. 6b, there is no influence of the external force F. To smoothly extend the knee, the torque $\tau_k$ less than that in the stance phase is output in the extension direction.

Torque output in the extension direction is common to the stance phase and the swing phase. Therefore, the knee is controlled using the angle in the fully extended position as a target angle. Since the stance phase needs a large torque and the swing phase needs a small torque, gains for flexion and gains for extension are set such that the gains for flexion differ from those for extension, and switching between the gains is performed based on a current rotation direction of the knee. A command torque in the stair descending action is given by Expression (4) as follows. This method of calculating a command torque is the same as that in the stance phase of the level walking action.

[Math. 3]

$$\tau_{command} = K_P \cdot \left( \theta_{knee_{ref}} - \theta_{knee}(t) \right) - K_D \cdot \dot{\theta}_{knee} \cdots (4)$$

$$\begin{cases} K_P = K_{P\_fl}, K_D = K_{D\_fl} : \left( \dot{\theta}_{knee}(t) > 0 \right) \\ K_P = K_{P\_ex}, K_D = K_{D\_ex} : \left( \dot{\theta}_{knee}(t) \leq 0 \right) \end{cases}$$

$\tau_{command}$ is the command torque,

$\theta_{knee}(t)$ is the knee velocity,
$\dot{\theta}_{knee}(t)$ is the knee angular velocity,
$\theta_{knee_{ref}}$ is the knee target angle,
$K_P, K_D$ are the gains.

The gains $K_{P\_ft}$ and $K_{D\_ft}$ for flexion are set to large values. This causes the knee to gradually flex when the wearer puts his or her weight on the action-assist device in the stance phase. In addition, the gains $K_{P\_ex}$ and $K_{D\_ex}$ for extension are set to small values. This causes the knee to smoothly extend in the swing phase. Stair descending is achieved by repeating the stance phase and the swing phase.

[Switching between Tasks]

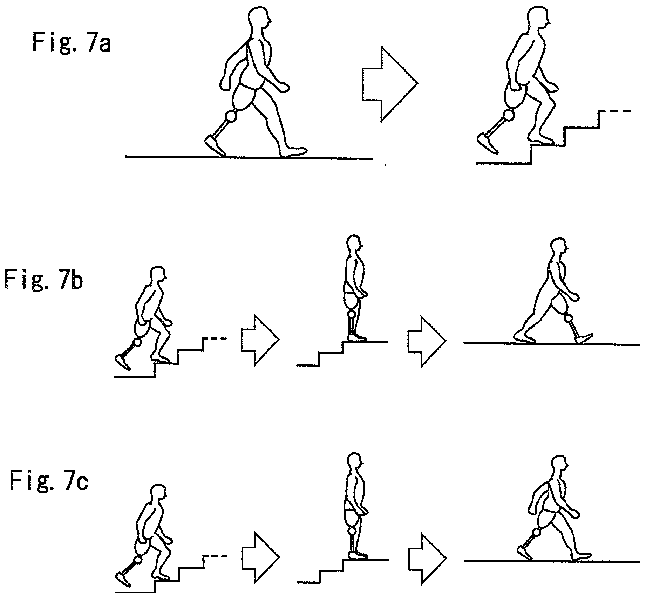

Figs. 7a to 7C illustrate exemplary circumstances in which task switching between the above-described level walking task and stair ascending task occurs. Fig. 7a illustrates a case where the wearer begins to ascend stairs with the healthy leg upon transition from the level walking task to the stair ascending task. Fig. 7b illustrates a case where the wearer begins to walk with the leg attached to the action-assist device (prosthetic leg) upon transition from the stair ascending task to the level walking task. Fig. 7c illustrates a case where the wearer begins to walk with the healthy leg, contrary to the case of Fig. 7b, upon transition from the stair ascending task to the level walking task.

For the case of Fig. 7a, a preliminary experiment on an able-bodied person is carried out in advance, and conditions for task switching are determined based on results of the preliminary experiment.

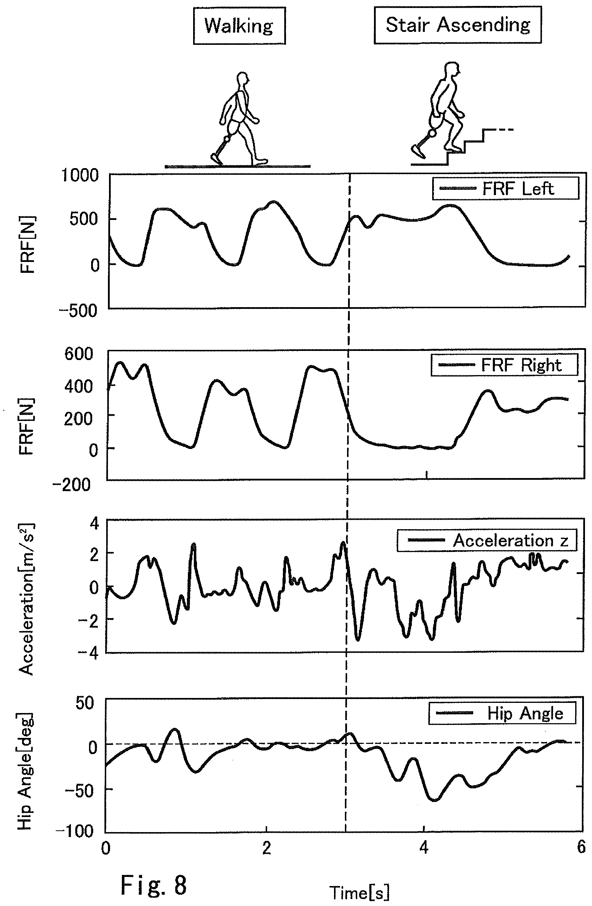

For example, in the preliminary experiment, one able-bodied person or subject equipped with the action-assist device attached to their right leg through a simulated upper-leg socket performed an action of walking on level ground as the level walking task and ascending stairs having a riser height of 180 mm such that the subject began to ascend the stairs with the healthy foot. Fig. 8 illustrates exemplary data obtained from this experiment. The horizontal axes of the graphs represent time [s] and the vertical axes thereof represent a floor reaction force (FRF Left) to the healthy foot, a floor reaction force (FRF Right) to the action-assist device, a vertical acceleration (Acceleration Z) obtained from the absolute angle sensor, and a hip angle (Hip Angle).

Since the subject began to ascend the stairs with the healthy foot, the healthy foot served as a supporting leg and the action-assist device served as a swing leg, so that FRF Left increased and FRF Right decreased. Furthermore, ascending one of the stairs with the healthy foot caused the vertical acceleration, resulting in a momentary increase in Acceleration Z. In addition, Hip Angle indicates that the hip joint adjacent to the action-assist device was at a downward angle relative to the vertical direction because the riser was in front of the action-assist device.

Threshold values of these parameters are set in consideration of the above-described characteristics. When these conditions are satisfied, the walking task is switched to the stair ascending task.

At the beginning of human walking motion, a large floor reaction force is generated at the toe of the foot opposite to or behind the first foot. For the case of Fig. 7b, therefore, a state where a large floor reaction force to the healthy foot (greater than or equal to a predetermined value) is generated and a state where the leg adjacent to the action-assist device is moving in a flexion direction are set to task switching conditions. For the case of Fig. 7c, a state where a large floor reaction force to the action-assist device (greater than or equal to the predetermined value) is generated and a state where the leg adjacent to the action-assist device is moving in the extension direction are set to task switching conditions.

The torque control for the stance phase of the stair descending task is the same as that for the stance phase of the level walking task. Stair descending is achieved by using the stance phase of the walking task. This eliminates the need for switching between the level walking task and the stair descending task.

As described above, in the action-assist device according to the present embodiment, an angular velocity is calculated based on an absolute thigh angle detected by the absolute angle sensor in the swing phase of the level walking task, and the angular velocity is used to flex the knee in conjunction with the hip joint. This achieves level walking similar to that of an able-bodied person.

In the flexion phase of the stair ascending task, the target knee angle for the first step is calculated based on the link lengths of the upper and lower legs of the wearer P by using the inverse kinematics so that the toe can pass over a step corresponding to the upper limit of a riser height of stairs determined in the Building Standards Law. The knee is flexed based on the target knee angle. When the flexion angle of the knee reaches the target angle, or alternatively when a biopotential signal from the extended leg is greater than or equal to the predetermined value, the flexion phase shifts to the foot flat phase. A target knee angle at which the bottom of the foot portion is in the flat position is calculated based on the angle of the thigh obtained from the absolute angle sensor. The knee is flexed based on the target knee angle. When the weight put on the foot portion 18 of the action-assist device reaches a predetermined value, the foot flat phase shifts to the pull-up phase. The knee is flexed based on the angle in the fully extended position as a target value. When the foot portion 18 of the action-assist device leaves a tread, the pull-up phase shifts to the flexion phase.

Controlling the knee based on the above-described target knee angles achieves step-over-step stair ascending.

In the stair descending task, since the gains of drive torques for flexion are set to be greater than those for extension, the knee is gradually flexed in the stance phase and the knee joint is smoothly extended in the swing phase. Thus, step-over-step stair descending is achieved.

Switching between the level walking task and the stair ascending task is performed based on, for example, floor reaction forces and the hip angle. The same torque control is performed in each of the stance phase of the stair descending task and that of the level walking task, such that stair descending is achieved by using the stance phase of the level walking task. Thus, seamless switching between the three tasks, namely, level walking, stair ascending, and stair descending, can be performed in accordance with the motion of the wearer P without using a special device, such as a switch. This achieves walking similar to that of an able-bodied person.

The wearable action-assist device according to the present embodiment may be a prosthesis-mounted action-assist device to be attached to a commercially available prosthesis (prosthetic leg). In this case, for example, components of the commercially available prosthetic leg function as an upper leg frame, a lower leg frame, a foot portion, and a joint rotatably connecting the upper and lower leg frames. The actuator 20 is connected to the upper and lower leg frames and is fixed such that its rotation axis coincides with, or is concentric with the center of rotation of the joint. The prosthesis-mounted action-assist device includes the actuator 20 of an external type, the floor reaction force sensors, the biological signal detection sensor, the absolute angle sensor, and the controller 40.

[Experiments on Able-bodied Person]

Experiments for Tasks

Experiments were carried out on an able-bodied person or subject equipped with the action-assist device through a simulated upper-leg socket used instead of the socket 10. The level walking action, the stair ascending action, and the stair descending action of the subject with the action-assist device and those of the subject without the action-assist device were experimented. The subject without the action-assist device walked at a comfortable speed. The subject with the action-assist device walked at the comfortable speed and walked at a maximum speed. Each of the following experiments was carried out five times under each of the above-described three conditions, and the results of the experiments were compared. In the present experiments, 14 stairs having a riser height of 180 mm, a tread width of 1400 mm, and a tread depth of 280 mm were used for stair ascending and descending.

(1) Level Walking Action Experiment

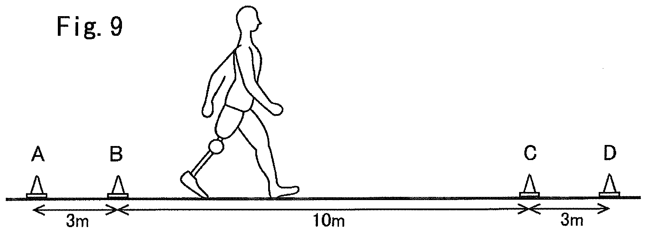

In the level walking action experiment, 10-m walking was tested. The subject walked from A point to D point of a path illustrated in Fig. 9. The time required to walk 10 m from B point to C point was measured. Each of a section from A point to B point and a section from C point to D point was a 3-m section for acceleration or deceleration.

(2) Stair Ascending Action Experiment

In the stair ascending action experiment, the time required to ascend ten stairs in total from the first stair to the eleventh stair was measured.

(3) Stair Descending Action Experiment

In the stair descending action experiment, the time required to descend ten stairs in total from the twelfth stair to the second stair was measured.

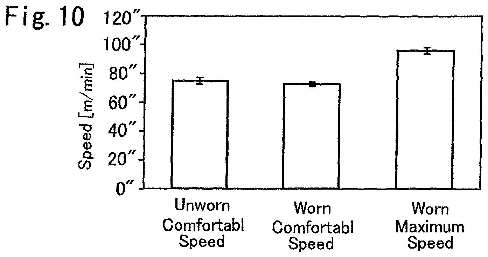

In the level walking action experiment, the stair ascending action experiment, and the stair descending action experiment, it was found that these actions were achieved by the torque control processes in the above-described embodiment. As regards the stair ascending and descending actions, it was found that step-over-step stair ascending and descending actions similar to those of an able-bodied person were achieved. Figs. 10, 11, and 12 illustrate speeds as results of the level walking action experiment, those of the stair ascending action experiment, and those of the stair descending action experiment, respectively.

Fig. 10 demonstrates that the comfortable speed obtained by using the action-assist device was substantially equal to that of the able-bodied subject. The maximum speed obtained by using the action-assist device was 1.3 times as high as that of the able-bodied subject. As a result, a leg amputee using the action-assist device can walk on level ground at a speed substantially equal to that of an able-bodied person.

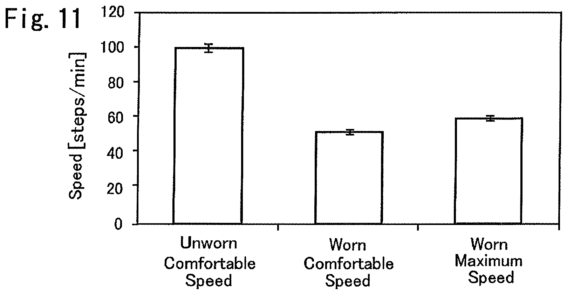

Fig. 11 demonstrates that in the stair ascending action, both the comfortable speed and the maximum speed obtained by using the action-assist device were lower than the comfortable speed of the able-bodied subject. The maximum speed obtained by using the action-assist device was 61% of the comfortable speed of the able-bodied subject. As a result, a leg amputee using the action-assist device can ascend stairs at a speed that is about 60% of the comfortable speed of an able-bodied person. The reason why the speeds obtained by using the action-assist device in the stair ascending action were lower than the speed of the able-bodied subject may be that the action-assist device including a fixed angle generated a trajectory so that the toe did not touch a riser, and thus had to trace a larger trajectory than an able-bodied subject.

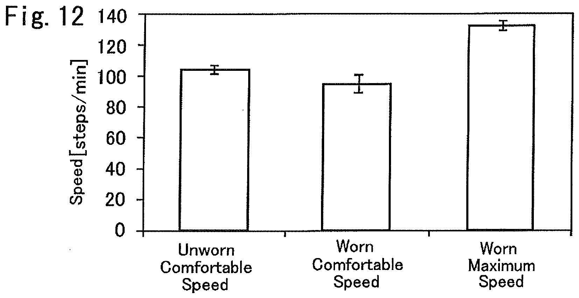

Fig. 12 demonstrates that in the stair descending action, the comfortable speed obtained by using the action-assist device was substantially equal to that of the able-bodied subject. The maximum speed obtained by using the action-assist device was 1.3 times as high as the comfortable speed of the able-bodied subject. As a result, a leg amputee using the action-assist device can descend stairs at a speed substantially equal to that of an able-bodied person.

Task Switching Experiment

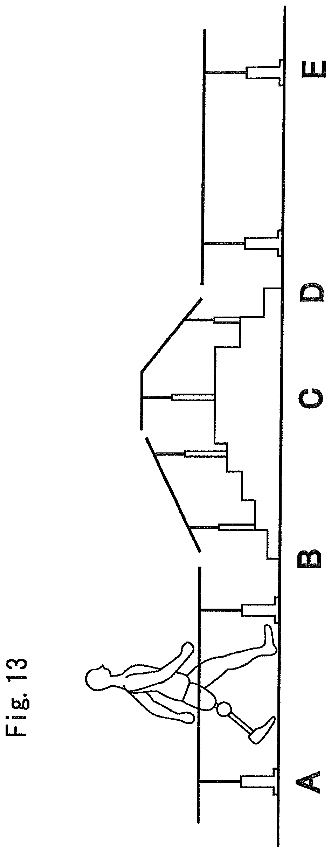

Task switching based on circumstances was experimented under conditions where one able-bodied subject provided with the action-assist device walked on a path that required the level walking action, the stair ascending action, and the stair descending action as illustrated in Fig. 13. The experiment used a series of steps including five steps on one side and three steps on the other side. The five steps had a riser height of 120 mm and the three steps had a riser height of 200 mm. In Fig. 13, the subject walked from A point, ascended the steps in B-C section, descended the steps in C-D section, and walked to E point. After that, the subject returned to A point on the same path.

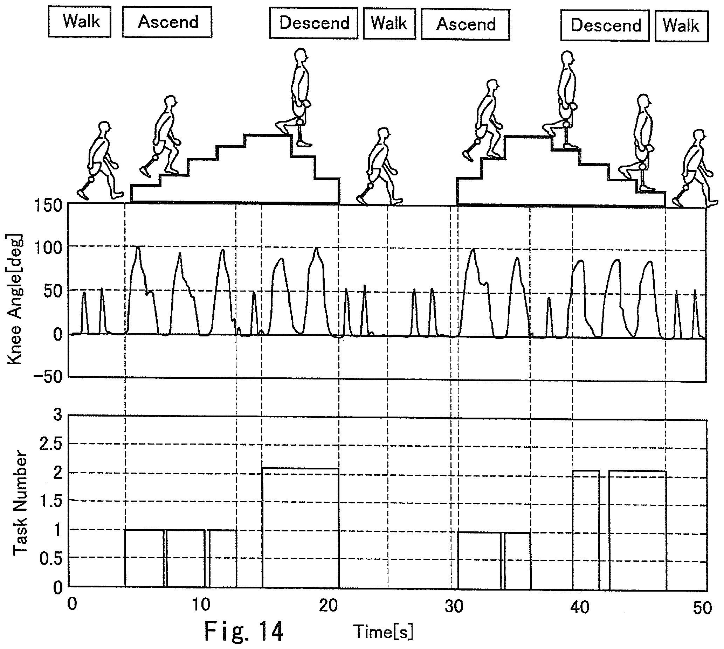

Fig. 14 illustrates results of this task switching experiment. Fig. 14 illustrates action states, knee angles of the action-assist device, and task numbers. The task number 0 indicates the level walking task, the task number 1 indicates the stair ascending task, and the task number 2 indicates the stair descending task. It was found that the task switching control according to the above-described embodiment achieved smooth task switching based on circumstances in accordance with an estimated motion intention of the wearer without using any special device, such as a switch.

[Experiments on Leg Amputee]

Experiments were carried out in cooperation with one leg amputee. The experiment cooperator was a male in his fifties and underwent an above-knee amputation of his right leg and a below-knee amputation of his left leg because of a traffic accident. He could voluntarily exert fore with his right leg because part of a group of muscles necessary for knee flexion and extension remained in the right leg. In the experiments, the action-assist device was attached to his right leg and a commercially available below-knee prosthetic leg was attached to his left leg. He had used prosthetic legs for ten years. He was usually able to walk on level ground by using an intelligent above-knee prosthetic leg while flexing and extending the knee of the prosthetic leg.

Since the action-assist device uses a floor reaction force alone as information about a healthy foot, this device can be applied not only to a patient with an above-knee amputation of one leg but also to the experiment cooperator with amputations of both legs.

Biopotential Measurement Experiment

Biopotential signals necessary for control of the action-assist device were measured for the knee adjacent to the right leg. The experiment cooperator was instructed to exert force so as to flex and extend the knee. Electrodes for biopotential measurement were attached to the surface of the skin over the muscle tensed at that time. While the action-assist device performed biopotential measurement, voluntary control based on the following Expression (5) was performed as feedback to the experiment cooperator.

[Math. 4]

$$\tau_{BES} = K_{fl} \cdot E_{fl} - K_{ex} \cdot E_{ex} \cdots (5)$$

In this expression, $\tau_{BES}$ denotes the command torque in voluntary control, $E_{fl}$ denotes the strength of a biopotential signal associated with flexion, $E_{ex}$ denotes the strength of a biopotential signal associated with extension, $K_{fl}$ denotes the gain for converting the biopotential signal associated with flexion into a knee command torque, and $K_{ex}$ denotes the gain for converting the biopotential signal associated with extension into a knee command torque. This voluntary control enabled the knee of the action-assist device to flex or extend based on a biopotential signal from the experiment cooperator.

In the present experiment, the experiment cooperator was instructed to flex and extend the knee. A change in waveform of the obtained biopotential signal and whether the knee of the action-assist device flexed and extended in response to instructions under the voluntary control were checked.

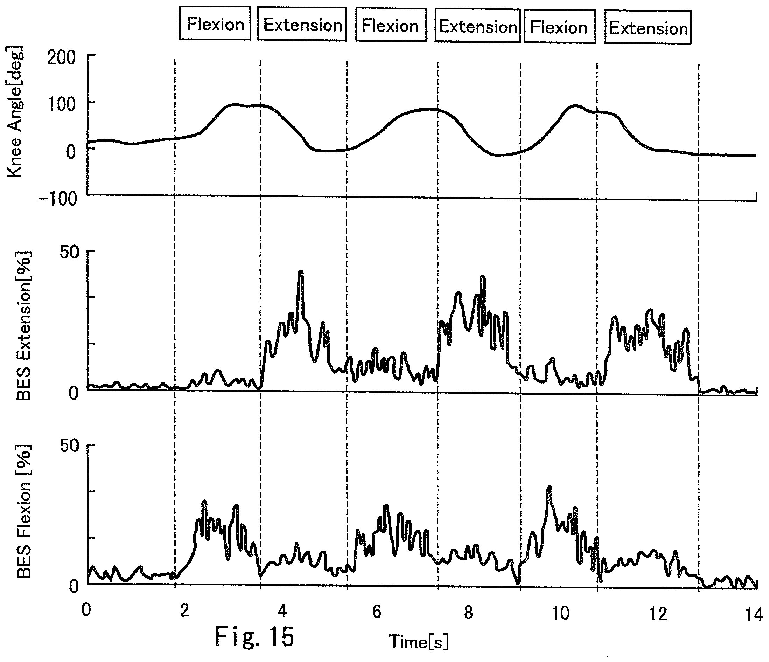

Fig. 15 illustrates results of the biopotential measurement experiment. In Fig. 15, Knee Angle denotes a change in angle of the knee of the action-assist device, BES Extension denotes the strength of the biopotential signal associated with extension, and BES Flexion denotes the strength of the biopotential signal associated with flexion. To obtain the results of biopotential signal measurement, the 100% MVC method was used.

The biopotential signals generated in response to the flexion and extension instructions were obtained. Fig. 15 demonstrates that voluntary control enabled the knee of the action-assist device to alternately flex and extend and the biopotential signals associated with flexion were obtained separately from those associated with extension without simultaneous contraction. Furthermore, the following feedback was obtained: the experiment cooperator said that he was able to move the device like his own leg and he felt as if his leg was recovered.

As a result, it was found that voluntary biopotential signals were obtained from the experiment cooperator. In addition, it was found that the biopotential signals had signal strengths enough to control the action-assist device.

Level Walking Action Experiment

Level walking of the leg amputee with the action-assist device was experimented. In the present experiment, 10-m walking was tested in a manner similar to the above-described experiment on the able-bodied subject. The amputee walked from A point to D point on the path illustrated in Fig. 9. The time required to walk 10 from B point to C point was measured. Each of the section from A point to B point and the section from C point to D point was the 3-m section for acceleration or deceleration.

For comparison, 10-m walking using the action-assist device and 10-m walking using the prosthetic leg used in daily life were experimented.

The experiment cooperator was instructed to walk using the action-assist device at a comfortable speed and walk using the prosthetic leg used in daily life at a comfortable speed. After sufficient practice and control parameter adjustment, the experiment was carried out.

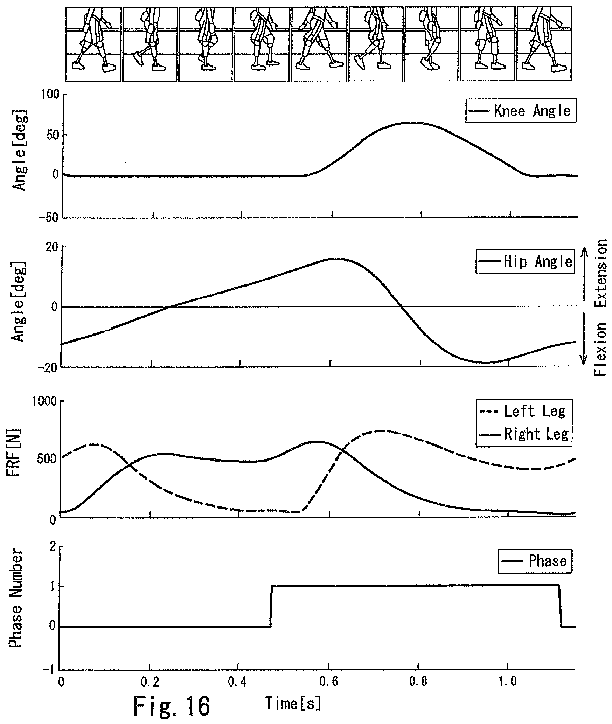

The 10-m walking experiment revealed that the leg amputee was able to walk on level ground using the action-assist device. Fig. 16 illustrates knee angles, hip angles, floor reaction forces to both legs, and phase numbers of one gait cycle in the use of the action-assist device. It was found that the leg amputee was able to walk while flexing and extending the knee and switching between the stance phase and the swing phase.

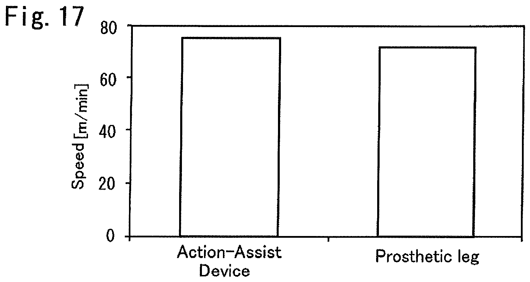

Fig. 17 illustrates speeds as results of the present experiment, namely, a speed calculated based on the time required to walk 10 m using the action-assist device and a speed calculated based on the time required to walk 10 m using the prosthetic leg used in daily life. The result associated with the action-assist device is 39 [s] and the result associated with the prosthetic leg used in daily life is 38 [s]. Fig. 17 demonstrates that the walking speed obtained by using the action-assist device was substantially equal to the speed obtained by using the prosthetic leg used in daily life.

The present experiment revealed that the leg amputee using the action-assist device was similarly able to walk on level ground. Since the speed substantially equal to that obtained by using the prosthetic leg used in daily life was achieved, a walking speed that may be used in daily life can be achieved.

Stair Ascending and Descending Action Experiments

Stair ascending and stair descending of the leg amputee with the action-assist device were experimented. The present experiments used the same stairs as those in the above-described experiments on the able-bodied subject. In the stair ascending action experiment, the time required to ascend ten stairs in total from the first stair to the eleventh stair was measured. In the stair descending action experiment, the time required to descend ten stairs in total from the twelfth stair to the second stair was measured. A handrail on one side was available. For comparison, the time required for the stair ascending action and the time required for the stair descending action in the use of the prosthetic leg used in daily life were measured in addition to measurement in the use of the action-assist device. The experiment cooperator was instructed to walk at a comfortable speed when using the action-assist device as well as when using the prosthetic leg used in daily life. To experiment these actions using the prosthetic leg used in daily life, the experiment cooperator ascended and descended the stairs in the step-by-step manner because he usually did in this manner.

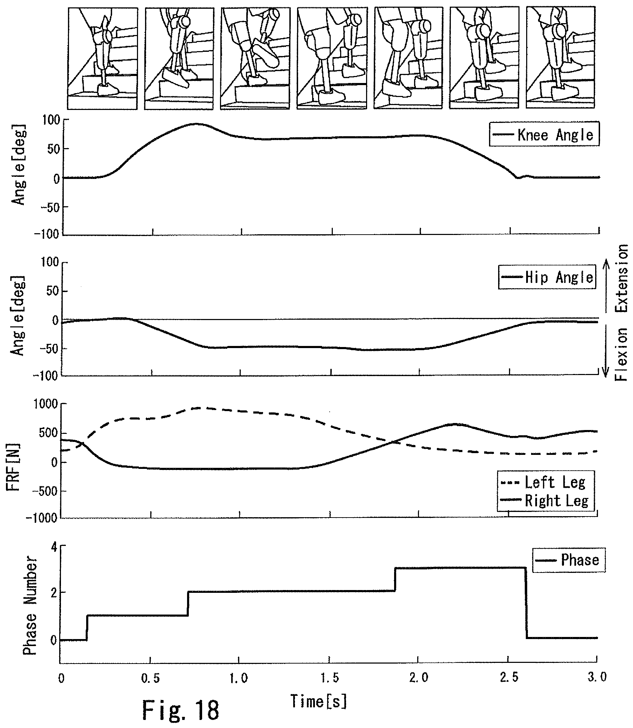

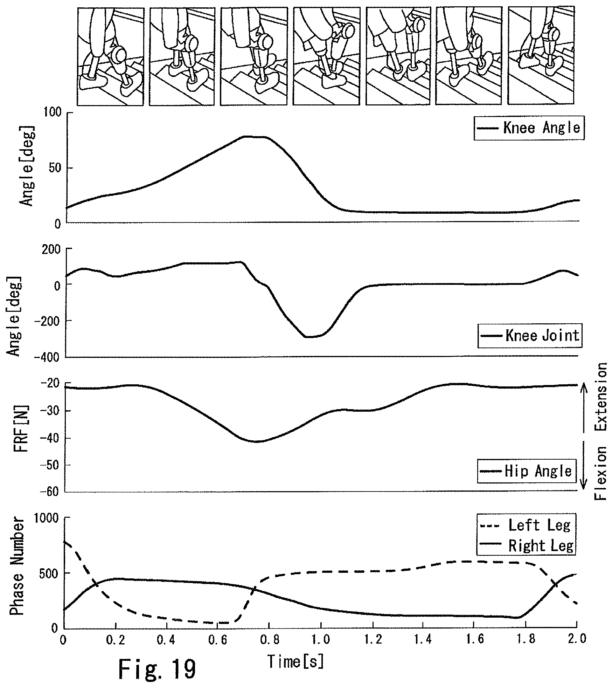

The present experiments revealed that the leg amputee was able to ascend and descend the stairs in the step-over-step manner using the action-assist device. Fig. 18 illustrates knee angles, hip angles, floor reaction forces to both legs, and phase numbers of one gait cycle in the stair ascending action experiment with the action-assist device. Fig. 19 illustrates knee angles, knee angular velocities, hip angles, and floor reaction forces to both legs of one gait cycle in the stair descending action experiment with the action-assist device.

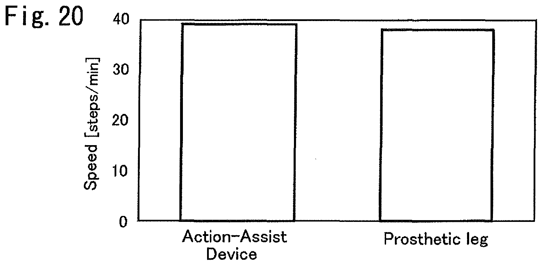

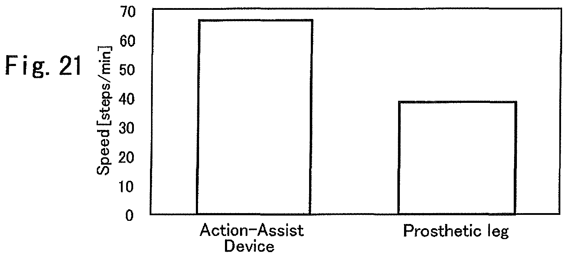

Figs. 20 and 21 illustrate speeds as results obtained by using the action-assist device and speeds as results obtained by using the prosthetic leg used in daily life. Fig. 20 illustrates the speeds as the results of the stair ascending action experiment. Fig. 21 illustrates the speeds as the results of the stair descending action experiment. A cadence [steps/min], indicating the number of stairs per minute, was calculated based on the measured time and the number of stairs where the amputee ascended or descended in each experiment.

Fig. 20 demonstrates that the speed obtained by using the action-assist device was substantially equal to the speed obtained by using the prosthetic leg used in daily life in the stair ascending action experiment.

Fig. 21 demonstrates that the speed obtained by using the action-assist device was 1.7 times as high as the speed obtained by using the prosthetic leg used in daily life in the stair descending action experiment.

Although the experiment cooperator's gait using a typical passive prosthetic leg was in the step-by-step manner, he was able to ascend the stairs in the step-over-step manner using the action-assist device in the stair ascending action experiment. Consequently, a gait similar to that of an able-bodied person was achieved. Furthermore, the following feedback was obtained: the experiment cooperator said that he was glad to ascend and descend the stairs. Consequently, a mental load imposed on the experiment cooperator during stair ascending can be relieved.

In the stair ascending action, the resultant speed obtained by the action-assist device was substantially comparable to that obtained by using the prosthetic leg used in daily life. One of the factors that caused the resultant speeds to be comparable to each other may be "habituation". This experiment was carried out once a week at earliest such that it took two hours per experiment. Each experiment involves, for example, an explanation of the details of the experiment, attachment of the device, biopotential measurement, and parameter adjustment. The time used for training was limited accordingly. Doing the training on a regular basis can enable an amputee to ascend stairs at a speed comparable to that of an able-bodied person. Another factor may be the fact that the experiment cooperator had amputations of both legs. In the stair ascending and descending actions, the ankles move more dynamically than those in the level walking action. Amputations of both legs cause the range of motion of an ankle of each prosthetic leg to be limited. Therefore, the resultant speeds of the experiment cooperator may be lower than those in the above-described experiments on the able-bodied subject. An amputee with an above-knee amputation of one leg can ascend stairs using the action-assist device at a speed close to the resultant speed in the experiment on the able-bodied subject.

In the stair descending action, the speed obtained by using the action-assist device was higher than that obtained by using the prosthetic leg used in daily life. A major factor may be that the gait associated with the prosthetic leg used in daily life was in the step-by-step manner but the gait associated with the action-assist device was different, namely, in the step-over-step manner. Furthermore, the limited range of motion of each ankle was overcome such that part of the foot portion corresponding to the arch of the foot was placed on the edge of a stair and the foot portion was rotated about the edge of the stair to achieve the functions of the ankle during stair descending.

Task Switching Experiment

Task switching based on circumstances was experimented under conditions where the leg amputee provided with the action-assist device walked on the path that required the level walking action, the stair ascending action, and the stair descending action as illustrated in Fig. 13. The experiment used the series of steps including five steps on one side and three steps on the other side. The five steps had a riser height of 120 mm and the three steps had a riser height of 200 mm. In Fig. 13, the leg amputee walked from A point, ascended the steps in B-C section, descended the steps in C-D section, and walked to E point. After that, the leg amputee returned to A point on the same path.

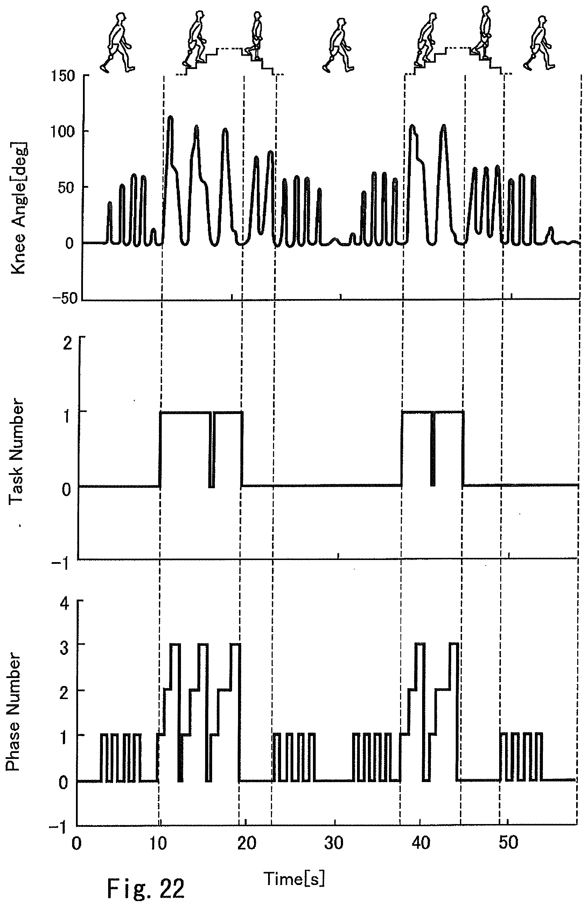

Fig. 22 illustrates results of this task switching experiment. Fig. 22 illustrates action states, knee angles of the action-assist device, task numbers, and phase numbers. The task number 0 indicates the level walking task and the task number 1 indicates the stair ascending task. Stair descending corresponds to a state in which both the task number and the phase number indicate 0. The reason is that the torque control in the stance phase of the level walking task was the same as that of the stair descending task as described above in the above-described embodiment.

Fig. 22 demonstrates that the experiment cooperator was able to walk on the experiment path that required level walking and stair ascending and descending. Furthermore, it was found that switching between the tasks was performed in accordance with circumstance without using any special device, such as a switch, and an intended action was achieved.

As described above, it was found that the leg amputee was able to independently walk on the path that required stair ascending and descending as well as level walking.

Walking Experiment for Indoor Use

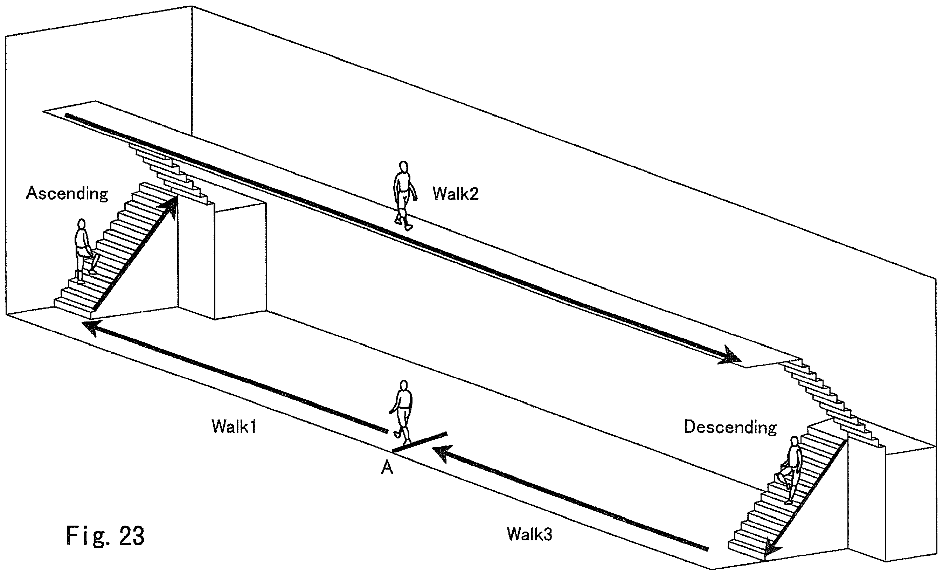

Whether the leg amputee was able to walk on a route that required the level walking action and the stair ascending and stair descending actions in an actual structure was investigated for indoor use in daily life. Fig. 23 is a schematic diagram of the interior of a structure. As regards a route for walking, the leg amputee started walking from A point, walked along the route including sections Walk 1, Ascend, Walk 2, Descend, and Walk 3 in that order in the structure, and returned to A point. The time required for the amputee to walk along the route was measured. For comparison, the leg amputee walked along the route with the prosthetic leg used in daily life in addition to walking with the action-assist device. In the experiment with the prosthetic leg used in daily life, the leg amputee ascended and descended the stairs in the step-by-step manner because he usually did in this manner.

Walking in the structure illustrated in Fig. 23 was accomplished with each of the action-assist device and the prosthetic leg used in daily life. In the use of the action-assist device, walking along the route for the experiment was accomplished such that stair ascending or descending was performed in the step-over-step manner.

The time required in the use of the action-assist device was 160 [s] and that in the use of the prosthetic leg used in daily life was 165 [s]. As a result, walking with the action-assist device was faster by 5 [s] than walking with the prosthetic leg used in daily life.

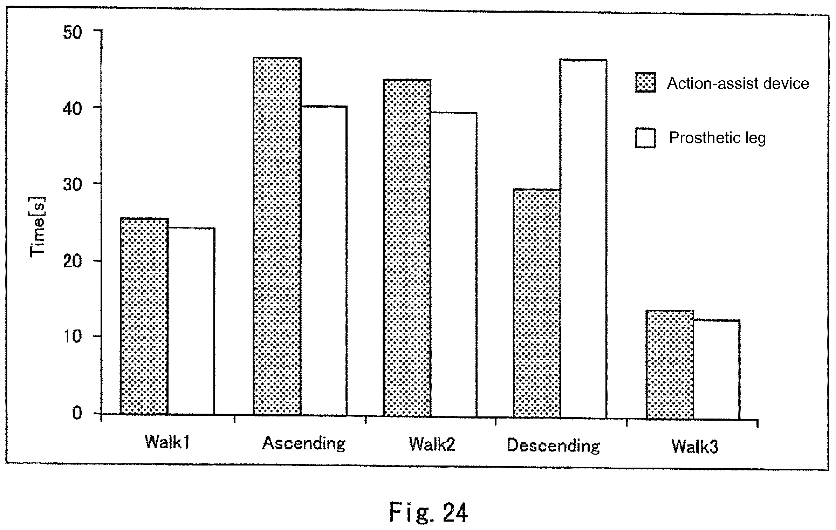

Fig. 24 illustrates the times required for walking with the action-assist device in the sections of the route and those with the prosthetic leg used in daily life in the sections of the route. As regards the sections Walk 1 and Walk 3, there was little difference between them. Concerning the sections Ascend and Walk 2, walking with the action-assist device was slower than that with the prosthetic leg used in daily life. As for the section Descend, walking with the action-assist device was faster than that with the prosthetic leg used in daily life.

The reason why the stair ascending action with the action-assist device was slower may be that the leg amputee had to direct his attention to all of the three tasks in the present experiment and took a little time to perform the stair descending task, which involves frequent switching between phases. If the leg amputee continues to conduct training along a route that requires level walking and stair ascending and descending, such as the route used in the present experiment, he can use the action-assist device unconsciously, resulting in an increase in moving speed.

While the present invention has been described in detail with reference to specific embodiments, it will be apparent to those skilled in the art that various modifications can be made without departing from the spirit and scope of the present invention.

The present application claims priority to Japanese Patent Application No. 2014-122482 filed June 13, 2014, which is hereby incorporated by reference herein in its entirety.

Description of Signs

| 10 | socket |
| 12 | upper leg frame |
| 14 | knee portion |
| 16 | lower leg frame |
| 18 | foot portion |
| 20 | actuator |
| 30 | waist belt |
| 40 | controller |
| 50 | battery |
| 102 | biopotential signal detecting means |
| 104 | joint angle detecting means |
| 106 | floor reaction force detecting means |
| 110 | control unit |
| 120 | drive signal generating means |

Claims

1. A prosthesis-mounted action-assist device that assists an action of a knee disposed between an upper leg frame and a lower leg frame of a prosthetic leg, the device comprising:

an actuator disposed outside the knee and connected to the upper leg frame and the lower leg frame, the actuator applying power to the prosthetic leg;
an absolute angle sensor detecting a hip angle relative to a vertical direction;
an angle sensor disposed in the actuator, the angle sensor detecting a knee angle;
a floor reaction force sensor detecting a floor reaction force to a wearer;
a data storage unit storing reference parameters of minimum motion units (phases) of action patterns of the wearer classified as tasks, each task including a series of phases; and
a control unit comparing the hip angle, the knee angle, and the floor reaction force with the reference parameters stored in the data storage unit to estimate a phase of a task of the wearer and generating an autonomous command signal for causing the actuator to produce power for the phase.

2. The device according to Claim 1, wherein the control unit calculates a target knee angle based on lengths of the upper and lower leg frames by using inverse kinematics to allow a toe of the prosthetic leg to pass over a step having a predetermined height in a flexion phase of a stair ascending task, and generates the autonomous command signal based on a difference between the target knee angle and the knee angle detected by the angle sensor.

3. The device according to Claim 1 or 2, wherein the control unit calculates a target knee angle based on the hip angle to cause a bottom of a foot portion to be in a flat position in a foot flat phase of a stair ascending task, and generates the autonomous command signal based on a difference between the target knee angle and the knee angle detected by the angle sensor.

4. The device according to Claim 2, wherein the control unit causes transition from the flexion phase to a foot flat phase when the knee angle detected by the angle sensor reaches the target knee angle.

5. The device according to any one of Claims 1 to 4,
wherein the control unit generates the autonomous command signal based on a product of a gain and a difference between the knee angle detected by the angle sensor and a knee angle in a fully extended position in a stance phase of a stair descending task or a level walking task, and
wherein a gain for flexion is greater than a gain for extension.

6. The device according to any one of Claims 1 to 5, wherein the control unit calculates a hip angular velocity based on the hip angle in a swing phase of a level walking task, and generates the autonomous command signal based on a product of a gain and a hip angular velocity to be obtained a predetermined time before.

7. The device according to any one of Claims 1 to 6, wherein the control unit performs switching between a level walking task and a stair ascending task based on the hip angle, the knee angle, and the floor reaction force.

8. The device according to Claim 7, wherein the control unit performs switching from the level walking task to the stair ascending task based on a floor reaction force to a healthy leg, a floor reaction force to the prosthetic leg, a vertical acceleration obtained from the absolute angle sensor, and the hip angle.

9. The device according to Claim 7 or 8, wherein the control unit performs switching from the stair ascending task to the level walking task when a floor reaction force to a healthy leg is at or above a predetermined value and the knee is moving in a flexion direction.

10. The device according to any one of Claims 7 to 9, wherein the control unit performs switching from the stair ascending task to the level walking task when a floor reaction force to a healthy leg is at or below a predetermined value and the knee is moving in an extension direction.

11. The device according to any one of Claims 1 to 10, further comprising:

   a biological signal sensor detecting a biological signal in a thigh of the wearer,
   wherein the control unit causes transition from a flexion phase to a bottom flat phase when the biological signal detected from the thigh during extension by the biological signal sensor has a strength at or above a predetermined value in a stair ascending task.

12. The device according to Claim 11,
wherein the control unit generates a voluntary command signal for causing the actuator to produce power based on the biological signal, and combines the voluntary command signal and the autonomous command signal to generate a combined command signal, and
wherein the actuator is driven by a drive current generated based on the combined command signal.

13. The device according to Claim 12,
wherein the data storage unit stores a combination ratio of the voluntary command signal to the autonomous command signal set for each of the phases of the tasks, and
wherein the control unit combines the voluntary command signal and the autonomous command signal in accordance with the combination ratio for an estimated phase to generate a combined command signal.

**14.** A wearable action-assist device comprising:

an upper leg frame having a first end connected to a socket to be attached to a thigh of a wearer;
a knee portion having a first side connected to the upper leg frame;
a lower leg frame having a first end connected to a second side of the knee portion;
a foot portion connected to a second end of the lower leg frame;
an actuator disposed in the knee portion, the actuator producing a driving force to rotate the upper leg frame or the lower leg frame;
an angle sensor disposed in the knee portion, the angle sensor detecting a knee angle;
an absolute angle sensor disposed in the upper leg frame, the absolute angle sensor detecting a hip angle relative to a vertical direction;
a floor reaction force sensor disposed in the foot portion, the floor reaction force sensor detecting a floor reaction force to the wearer;
a data storage unit storing reference parameters of minimum motion units (phases) of action patterns of the wearer classified as tasks, each task including a series of phases; and
a control unit comparing the hip angle, the knee angle, and the floor reaction force with the reference parameters stored in the data storage unit to estimate a phase of a task of the wearer, and generating an autonomous command signal for causing the actuator to produce power for the phase.

Fig. 1

Fig. 2

# Fig. 3

| Right leg | Standing | Swing | Landing | Support | | Swing |
|---|---|---|---|---|---|---|
| Left leg | Standing | Support | | Swing | Landing | Support |

Fig. 4a

Fig. 4b

Fig.5

Fig. 6a

Fig. 6b

Fig. 7a

Fig. 7b

Fig. 7c

Fig. 8

Time[s]

Fig. 9

A     B                                                              C     D

3m                              10m                              3m

Fig. 10

Fig. 11

Fig. 12

Fig. 13

A          B          C          D          E

Fig. 14

Fig. 15

Fig. 16

## Fig. 17

Fig. 18

Fig. 19

Fig. 20

Bar chart. Y-axis: Speed [steps/min], scale 0, 10, 20, 30, 40. X-axis categories: Action-Assist Device (approximately 39), Prosthetic leg (approximately 38).

Fig. 21

Fig. 22

Walk2

Walk1

Walk3

Ascending

Descending

A

Fig. 23

Fig. 24

## INTERNATIONAL SEARCH REPORT

| International application No. |
| --- |
| PCT/JP2015/067034 |

**A. CLASSIFICATION OF SUBJECT MATTER**
*A61F2/70*(2006.01)i, *A61F2/72*(2006.01)i, *B25J11/00*(2006.01)i

According to International Patent Classification (IPC) or to both national classification and IPC

**B. FIELDS SEARCHED**

Minimum documentation searched (classification system followed by classification symbols)
A61F2/70, A61F2/72, B25J11/00

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 2009-60946 A (University of Tsukuba),<br>26 March 2009 (26.03.2009),<br>paragraphs [0013] to [0015], [0054] to [0086];<br>fig. 1 to 3, 7 to 12<br>(Family: none) | 1,14<br>2-4,7,9-10<br>5-6,8,11-13 |
| Y<br>A | Takumi TAKETOMI et al., Stair Ascent assistance<br>for Cerebral Palsy with Robot Suit HAL, System<br>Integration (SII), 2012 IEEE/SICE International<br>Symposium on, 2012, 331-336 | 2-4,7,9-10<br>1,5-6,8,<br>11-14 |
| Y<br>A | Kenta SUZUKI et al., Intention-based walking<br>support for paraplegia patients with Robot Suit<br>HAL, Advanced Robotics, 2007, Vol. 21, No. 12,<br>1441-1469 | 2-4,7,9-10<br>1,5-6,8,<br>11-14 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    14 July 2015 (14.07.15) | Date of mailing of the international search report<br>    28 July 2015 (28.07.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |

Form PCT/ISA/210 (second sheet) (July 2009)

**INTERNATIONAL SEARCH REPORT**

International application No.

PCT/JP2015/067034

C (Continuation). DOCUMENTS CONSIDERED TO BE RELEVANT

| Category* | Citation of document, with indication, where appropriate, of the relevant passages | Relevant to claim No. |
|---|---|---|
| A | JP 2010-17390 A (University of Tsukuba),<br>28 January 2010 (28.01.2010),<br>entire text; all drawings<br>(Family: none) | 1-14 |
| A | JP 2013-510603 A (Otto Bock Healthcare<br>Products GmbH),<br>28 March 2013 (28.03.2013),<br>entire text; all drawings<br>& US 2012/0232673 A1     & WO 2011/057790 A1<br>& DE 102009052888 A1 | 1-14 |

Form PCT/ISA/210 (continuation of second sheet) (July 2009)

## REFERENCES CITED IN THE DESCRIPTION

### Patent documents cited in the description

- JP 2009060946 A
- JP 2014122482 A